# FASCICULE DE BREVET EUROPEEN

(11) **EP 1 205 752 B1**
(45) Date de publication et mention de la délivrance du brevet: **06.06.2007**
(21) Numéro de dépôt: 01811068.4
(22) Date de dépôt: 05.11.2001
(51) Int. Cl.: G01N 33/14

(54) **Appareil de mesure de paramètres physiques d'un mout de raisin et du vin obtenu à partir de ce mout**
Vorrichtung zum messen physikalischer Parameter von Traubenmost und des daraus erhaltenen Rotweins
Apparatus to measure physical parameters of a grape-must and the wine obtained from the must

(30) Priorité: 09.11.2000 FR 0014720
(43) Date de publication de la demande: 15.05.2002
(73) Titulaire: Ecole d'Ingénieurs de Genève, 1202 Genève (CH)
(72) Inventeur: Brun, Daniel, 1218 Grand-Saconnex (CH)
(74) Mandataire: Nithardt, Roland

(56) Documents cités:
- WO-A-99/53312
- DE-A- 2 243 397
- DE-A- 3 609 156
- DE-A- 19 642 229
- FR-A- 1 601 654
- US-A- 4 547 070
- DATABASE WPI Section Ch, Week 200005 Derwent Publications Ltd., London, GB; Class D16, AN 2000-060381 XP002176390 & PT 102 177 A (CETO CENT CIENCIAS & TECNOLOGIAS OPTICAS), 31 décembre 1999 (1999-12-31)
- DATABASE WPI Section Ch, Week 198017 Derwent Publications Ltd., London, GB; Class D16, AN 1980-30673C XP002174882 & SU 681 372 A (KRASD POLY), 25 août 1979 (1979-08-25)
- DATABASE WPI Section Ch, Week 198031 Derwent Publications Ltd., London, GB; Class E17, AN 1980-54866C XP002174883 & SU 700 835 A (AS USSR BIOCHEM), 30 novembre 1979 (1979-11-30)

## Description

La présente invention concerne un appareil de mesure de paramètres physiques d'un moût de raisin et du vin obtenu à partir de ce moût, comportant des moyens pour effectuer une mesure de la turbidité d'un échantillon dudit moût par néphélémétrie.

Plusieurs éléments chimiques entrent dans la composition du vin obtenu par fermentation de jus de raisin frais. Les principales substances sont les alcools, principalement l'éthanol et le glycérol, des acides, des polyphénols, qui confèrent à chaque vin un goût particulier. Les propriétés organoleptiques des vins qui regroupent leurs caractéristiques sensitives, visuelles et olfactives permettent de les différencier, de les reconnaître et de préciser leur qualité. Un examen visuel permet d'avoir une appréciation sur la couleur et sur la limpidité du vin tandis qu'un examen gustatif permet de reconnaître plusieurs goûts et de définir par exemple l'acidité ou le moelleux, l'astringence et la teneur en alcool.

Afin pouvoir contrôler et modifier l'évolution de toutes ces caractéristiques lors de la vinification, la fermentation du moût exige l'exécution d'un certain nombre d'analyses oenologiques. Certaines analyses de base nécessitent peu de matériel de laboratoire et sont accessibles au plus grand nombre des vignerons. Toutefois, l'évolution de la vinification exige parfois de connaître plus de paramètres analytiques sur le vin. Ces analyses ne sont alors plus à la portée des producteurs peu équipés. Les échantillons des vins à analyser doivent alors être dirigés vers des laboratoires spécialement équipés pour de telles analyses et possédant un équipement adéquat dont la précision élevée n'est pas nécessaire dans ces cas là et qui sont chers à l'achat.

Cette manière de pratiquer comporte un inconvénient qui est lié au temps de transport des échantillons et au temps de retour des informations. Ces délais ne sont pas toujours compatibles avec les exigences oenologiques qui devraient être satisfaites immédiatement pour ne pas nuire à l'évolution de la fermentation. Dans ces situations, la dépendance du vigneron est liée à la performance de son équipement qui est pratiquement toujours trop faible, étant donné les coûts souvent prohibitifs de certains appareils de laboratoire.

On connaît, notamment, par la publication allemande DE 36 09 156 un procédé de mesure en continu de petits troubles dans des liquides et en particulier dans la bière. Ce procédé est basé sur l'utilisation de deux sources lumineuses modulées à des fréquences différentes et d'un récepteur de rayonnement unique. La comparaison des deux fréquences, l'une servant de valeur de référence permet de contrôler en continu sur une chaîne de production la turbidité du liquide fabriqué. Ce procédé n'autorise que le contrôle d'un seul paramètre de fabrication.

DE 296 16 677 U concerne un dispositif de mesure de la turbidité ou de la couleur de boissons comme du vin ou de la biere. Le procédé objet de la publication PT 102177 a pour but d'obtenir un vin de qualité constante lorsqu'il est obtenu à partir d'un mélange de vins provenant d'une même vigne mais ayant des caractéristiques différentes dues aux conditions climatiques. On obtient une constante de ces caractéristiques par un contrôle spectrocolorimétrique du mélange et la détermination graphique du pourcentage et de la concentration des composants de ce mélange. Le matériel utilisé pour ce type de contrôle et d'enregistrement est coûteux et encombrant.

La publication DE 196 42 229 concerne la composition d'un réactif chimique utilisé pour la mesure par néphélémétrie des tanins dans les boissons telles que les bières, les vins ou les jus de fruits et les conditions dans lesquelles ce réactif peut être utilisé.

La présente invention se propose de pallier les inconvénients de l'art antérieur en offrant un appareil de mesure d'encombrement réduit, facilement transportable, dont le coût est peu élevé et qui réunit les fonctions rencontrées habituellement dans deux appareils qu'il faut acheter séparément, à savoir un néphélomètre qui permet de contrôler la turbidité des moûts et des vins, en vue d'une bonne fermentation alcoolique, et un colorimètre qui permet de réaliser les analyses oenologiques nécessaires à l'élaboration d'un vin.

Cet appareil de mesure tel que défini en préambule est caractérisé en ce qu'il comporte en outre des moyens pour effectuer au moins une analyse par colorimétrie afin de contrôler la teneur de cet échantillon en au moins un composant prédéterminé et la couleur du vin obtenu après la fermentation dudit moût, lesdits moyens pour effectuer une analyse par colorimétrie étant associés aux moyens pour effectuer une mesure de la turbidité et logés dans un même boîtier

Dans la forme de réalisation préférée de l'invention, les moyens pour effectuer la mesure de turbidité comportent une source lumineuse associée à des moyens de détection. Cette source lumineuse peut être une source de lumière blanche ou de lumière monochromatique.

Les moyens pour effectuer des analyses par colorimétrie comportent avantageusement au moins une source lumineuse dont la longueur d'onde d'émission est fixe et prédéterminée en fonction du composant à mesurer, ladite au moins une source lumineuse étant associée à des moyens de détection.

De façon similaire, les moyens pour effectuer des mesures de la couleur du vin comportent au moins deux sources lumineuses de longueurs d'onde d'émission différentes, chaque longueur d'onde étant fixe et prédéterminée, lesdites sources lumineuses étant associées à des moyens de détection.

De façon avantageuse, l'échantillon à mesurer est disposé entre les sources lumineuses et les moyens de détection.

Dans toutes les formes de réalisation de l'appareil selon l'invention, l'échantillon à mesurer est disposé dans un logement ménagé au centre d'un puits de mesure de base carrée, ce puits étant pourvu sur deux de ses faces adjacentes d'un conduit de lumière ménagé entre ladite face et le logement central, l'axe longitudinal dudit conduit de lumière étant perpendiculaire au plan de la face dans laquelle il débouche, et les moyens de détection sont incorporés respectivement dans les deux autres faces et disposés dans l'alignement des conduits de lumière.

De préférence, les moyens de détection sont des photodiodes au silicium.

De façon avantageuse, les moyens de détection sont disposés à la sortie des conduits de lumière et à 90 degrés l'un de l'autre par rapport à l'échantillon à contrôler afin de permettre une mesure de la turbidité en mode axial et en quadrature.

Afin de pouvoir réunir deux fonctions, la source lumineuse pour la néphélémétrie et la au moins une source lumineuse pour les mesures de la teneur en composants d'une part, et les sources lumineuses pour la mesure de la couleur du vin d'autre part, sont disposées à l'entrée des conduits de lumière du puits de mesure et à 90 degrés les unes des autres par rapport à l'échantillon à contrôler.

De préférence, les sources lumineuses utilisées sont des diodes photoluminescentes.

Dans la forme de réalisation préférée, l'appareil de mesure comporte des moyens de traitement des signaux émis par les moyens de détection, lesdits moyens de traitement étant agencés pour rechercher les valeurs extrêmes mesurées par lesdits moyens de détection et associés à des moyens d'affichage de ces valeurs mesurées.

Les composants du moût dont la teneur peut être contrôlée par l'appareil sont le fer, le glycérol, l'acide tartrique et l'acétaldéhyde.

L'appareil peut également comporter des moyens agencés pour modifier l'intensité lumineuse des sources lumineuses en fonction des valeurs à mesurer.

La présente invention sera mieux comprise en référence à la description d'un exemple de réalisation préféré et des dessins annexés dans lesquels :

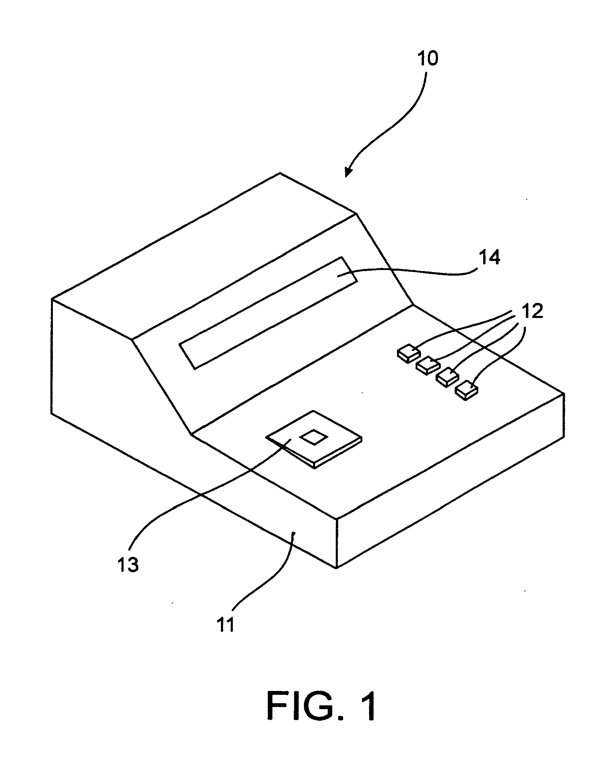

La figure 1 est une vue en perspective de l'appareil de mesure selon l'invention, et
La figure 2 est une représentation schématique des composants de l'appareil illustré par la figure 1.

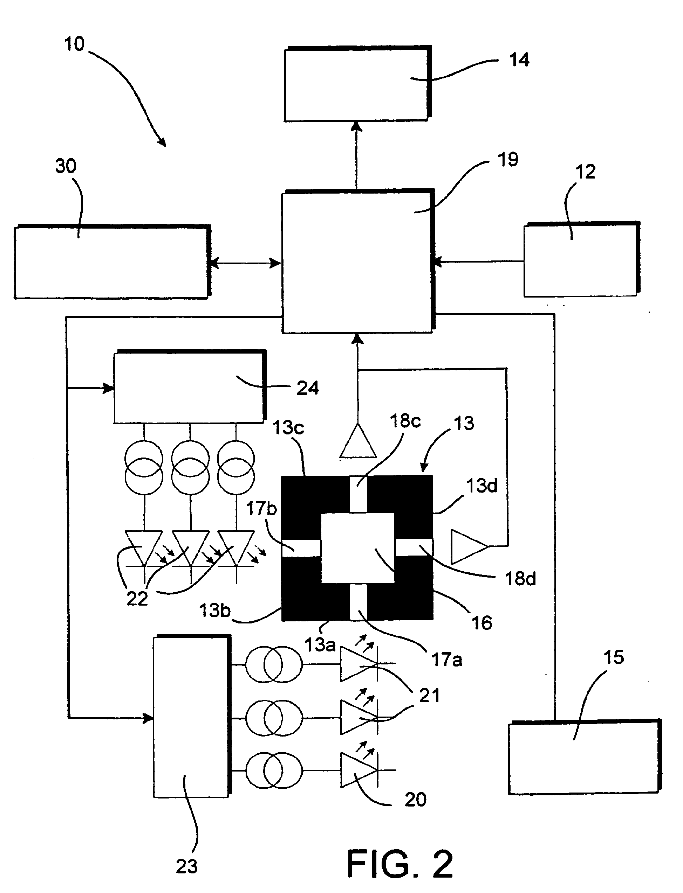

En référence aux figures, l'appareil de mesure 10 selon l'invention comporte un boîtier 11, destiné à loger les différents composants qui seront décrits ci-après en référence à la figure 2. Ce boîtier est pourvu sur sa face avant de touches de fonction 12, d'un logement destiné à recevoir un puits de mesure 13 dans lequel l'échantillon à mesurer est introduit, et d'un écran alphanumérique 14 permettant l'affichage des menus pouvant être utilisés et des résultats de mesure. Il est également équipé d'un logement et d'une prise (non représentés) agencés respectivement pour recevoir des moyens d'alimentation 15 qui peuvent être un jeu de piles bâtons ou un bloc externe à l'appareil, celui-ci étant alimenté en basse tension, c'est-à-dire une tension inférieure ou égale à 12 volts.

Le puits de mesure 13 se présente sous la forme d'un parallélépipède rectangle de base carrée réalisé par moulage en un matériau synthétique. Ce puits comporte en son centre un logement parallélépipédique 16 agencé pour recevoir une éprouvette en verre standardisée de 1 cm de côté contenant un échantillon du liquide à mesurer. Ce puits est également pourvu sur deux de ses faces adjacentes 13a, 13b d'un conduit de lumière respectivement 17a, 17b, sous la forme d'une ouverture cylindrique, dont l'axe longitudinal est perpendiculaire au plan de la face et qui débouche d'une part dans ladite face et d'autre part dans le logement 16. Les deux autres faces du puits, c'est-à-dire les faces 13c et 13d, sont pourvues de moyens de détection 18c et 18d incorporés dans lesdites faces et disposés respectivement dans l'alignement des conduits de lumière 17a et 17b. Ces moyens de détection sont reliés à des moyens de traitement sous la forme d'une unité centrale de traitement 19.

A l'entrée des conduits de lumière 17a et 17b sur les faces 13a et 13b l'appareil de mesure 10 comporte respectivement une source de lumière 20 qui peut être blanche ou monochromatique et au moins une source de lumière monochromatique 21, respectivement 22 dont la longueur d'onde est fixe et prédéterminée en fonction des paramètres à analyser. L'ensemble des sources lumineuses 20 et 21 est disposé à 90 degrés de l'ensemble formé par les sources lumineuses 22, de sorte que l'échantillon à mesurer, qui est disposé dans le logement 16, est situé d'une part entre les sources 20 et 21 et les moyens de détection 18c et 18d destinés aux mesures de turbidité et de certains composants du moût et d'autre part entre les sources 22 et le moyen de détection 18d pour les mesures de couleur du vin. Les sources lumineuses 20, 21 et 22 se présentent sous la forme de diodes électroluminescentes reliées à des circuits de commande 23 respectivement 24 agencés pour calibrer et asservir le courant qui les alimente. Ces circuits de commande sont pilotés par l'unité centrale de traitement 19.

Cette unité centrale de traitement 19, à laquelle sont également reliées les touches de fonction 12 et l'écran d'affichage 14, gère les signaux d'entrée qui sont des signaux analogiques amplifiés issus des moyens de détection 18c, 18d. Elle permet, à l'aide d'algorithmes et d'un logiciel appropriés, de rechercher les valeurs extrêmes mesurées par les moyens de détection. Ces valeurs mesurées sont délivrées à l'écran d'affichage 14 par l'intermédiaire des touches 12.

Ces touches de fonction 12, qui sont au nombre de quatre dans l'exemple décrit, permettent de sélectionner :
■ Le choix des menus liés à la fonction à utiliser
■ Les déplacements haut-bas dans le menu affiché
■ L'activation de la commande ou de la mesure demandée.

L'appareil de mesure 10 est en outre pourvu de ports de communication 30 permettant son possible raccordement à des unités périphériques telles qu'une imprimante ou un ordinateur portable. Il comporte également des moyens permettant de modifier l'intensité lumineuse des sources lumineuses en fonction des valeurs à mesurer.

Cet appareil de mesure, de par sa conception, permet ainsi d'effectuer, d'une part, la mesure de la turbidité d'un échantillon de liquide par néphélémétrie et, d'autre part, d'effectuer des analyses par colorimétrie afin de contrôler la teneur de cet échantillon en au moins un composant prédéterminé.

La néphélémétrie, qui consiste à mesure la transparence d'un liquide, est très utilisée lors des opérations de débourbage des moûts, en début de vinification. Il est donc important de pouvoir contrôler régulièrement la turbidité ou teneur en trouble de ce moût tout au long de l'élaboration du vin afin de pouvoir obtenir la turbidité idéale, valeur à laquelle toutes les qualités du vin sont mises en évidence. Cette valeur idéale varie d'un cépage à l'autre. Ainsi pour un moût de chasselas la turbidité idéale se situe autour de 100 NTU (Nephelometric Turbidity Unit) alors que pour un moût de chardonnay, cette valeur se situe plutôt autour de 150 à 200 NTU.

Ce type de mesure est également utilisé pour juger l'efficacité de la préfiltration des vins. Un vin préfiltré devrait se trouver au-dessous de 3 à 4 NTU pour passer facilement sur un filtre de finition. Si la turbidité dépasse ces valeurs, le filtre de finition se colmate rapidement, ce qui nécessite alors des interruptions de filtration pour changer les plaques filtrantes ou régénérer le filtre

Cette mesure de turbidité est effectuée très rapidement avec l'appareil selon l'invention. En effet elle consiste
■ à placer un échantillon du moût à contrôler directement prélevé des cuves dans le logement prévu à cet effet dans le puits de mesure,
■ à sélectionner la fonction «néphélémétrie» à l'aide de la touche de sélection de fonction,
■ à valider cette sélection à l'aide de la touche de validation,
■ à lire sur l'écran d'affichage la valeur directement en NTU de l'échantillon mesuré.

Pour cette première fonction de l'appareil, la source lumineuse 20, qui émet de la lumière blanche est enclenchée et, la mesure se faisant en mode axial et en quadrature, les moyens de détection 18c et 18d qui sont des photodiodes au silicium, fournissent un signal proportionnel à l'intensité lumineuse reçue à la sortie de l'échantillon. Ce signal est transmis après amplification à l'unité de traitement 19 et traité pour permettre d'obtenir sur l'écran 14 l'affichage direct de la valeur de la turbidité de l'échantillon.

Lorsque l'appareil est utilisé en tant que colorimètre, il permet en particulier de réaliser les analyses suivantes sur les moûts ou sur les vins obtenus à partir de ces moûts:
- analyse de l'acide tartrique en vue de procéder à une désacidification ou une acidification et de déterminer la stabilité physique du vin,
- analyse de l'acétaldéhyde pour juger ou comparer des méthodes de vinification,
- analyse du fer pour déterminer si un vin est en cours de "casse",
- analyse des alcools présents, notamment du glycérol,
- analyse de la couleur des vins rouges.

Lorsque l'on procède aux mesures relatives à la teneur du moût en un certain composant, le colorimètre travaillant par comparaison d'une couleur avec une couleur étalon de longueur d'onde prédéterminée, il est nécessaire d'établir au préalable une courbe d'étalonnage relative au composant mesuré sur base d'un échantillon calibré en laboratoire.

L'appareil tel que décrit est agencé pour mesurer en particulier la teneur en fer, en glycérol, en acide tartrique et en acétaldéhyde du moût de raisin.

A cet effet la source lumineuse 21 comprend, dans l'exemple représenté, trois photodiodes dont les longueurs d'onde sont respectivement de
470 nm pour la mesure du fer et du glycérol
520 nm pour la mesure de l'acide tartrique
570 nm pour la mesure de l'acétaldéhyde

Ces mesures sont réalisées séparément et, pour chaque composant, consiste
■ à définir le composant à mesurer,
■ à valider cette sélection à l'aide de la touche de validation,
■ à mettre l'appareil à zéro avec un échantillon d'eau pure,
■ à placer un échantillon du moût limpide et préalablement filtré dans le logement du puits de mesure,
■ à lire la valeur de l'absorption et, à l'aide de la courbe d'étalonnage préétablie, en déduire la teneur de l'échantillon en composant mesuré.

Pour cette seconde fonction de l'appareil, la photodiode de la source lumineuse 21 qui correspond au composant à mesurer étant enclenchée, le moyen de détection correspondant, qui est également une photodiode au silicium, fournit un signal proportionnel à l'intensité lumineuse reçue à la sortie de l'échantillon. Ce signal est transmis après amplification à l'unité de traitement 19 et traité pour permettre d'obtenir sur l'écran 14 l'affichage de la valeur de l'absorption par l'échantillon du rayonnement lumineux émis.

Les mesures de couleur, qui ne se font que sur un échantillon de vin rouge, ont pour but de connaître l'intensité de la couleur. Ces mesures sont obtenues par addition des absorptions à différentes longueurs d'ondes de la lumière émise par les sources lumineuses 22 et mesurées par le moyen de détection 18d. Ces sources 22 sont, dans l'exemple illustré, trois photodiodes dont les longueurs d'onde sont respectivement de 420, 520 et 620nm. Dans le but de simplifier l'appareil, dans l'exemple de réalisation représenté, la diode 22 qui émet une lumière ayant une longueur d'onde 520 nm est aussi utilisée comme source lumineuse 21 pour la mesure de l'acide tartrique contenu dans le moût.

Il est également possible de procéder avec l'appareil selon l'invention à des analyses telles que des analyses enzymatiques afin de déterminer en particulier la teneur en acide malique, acide qui se dégrade pendant la fermentation malo-lactique. Les teneurs en glucose et fructose (sucres de raisin) peuvent également être appréciées.

On peut également procéder à l'analyse des polyphénols pour établir des comparaisons entre les différentes techniques de vinification pouvant être utilisées pour des vins rouges.

La conception de cet appareil est avantageuse en ce qu'elle permet d'obtenir deux types de mesures distinctes avec un seul échantillon à mesurer, étant donné que cet échantillon est placé simultanément dans deux circuits de mesure pouvant être sélectionnés indépendamment l'un de l'autre. De plus, sa manipulation simple, son faible encombrement et son prix de revient peu élevé en font un appareil pouvant être facilement utilisé par les vignerons.

L'appareil selon l'invention peut également être utilisé pour mesurer d'autres composants d'un autre type de liquide en l'équipant de photodiodes ayant une longueur d'onde appropriée.

## Revendications

1. Appareil de mesure (10) de paramètres physiques d'un moût de raisin et du vin obtenu à partir de ce moût, comportant des moyens pour effectuer une mesure de la turbidité d'un échantillon dudit moût par néphélémétrie et en outre des moyens pour effectuer au moins une analyse par colorimétrie afin de contrôler la teneur de cet échantillon en au moins un composant prédéterminé et la couleur du vin obtenu après la fermentation dudit moût, **caractérisé en ce que** lesdits moyens pour effectuer une analyse par colorimétrie étant associés aux moyens pour effectuer une mesure de la turbidité et logés dans un même boîtier (11) facilement transportable.

2. Appareil de mesure selon la revendication 1, **caractérisé en ce que** les moyens pour effectuer la mesure de turbidité comportent une source lumineuse (20) associée à des moyens de détection (18c, 18d).

3. Appareil de mesure selon la revendication 2, **caractérisé en ce que** la source lumineuse (20) est une source de lumière blanche.

4. Appareil de mesure selon la revendication 2, **caractérisé en ce que** la source lumineuse (20) est une source de lumière monochromatique.

5. Appareil de mesure selon les revendications 1 et 2, **caractérisé en ce que** les moyens pour effectuer des analyses par colorimétrie comportent au moins une source lumineuse (21) dont la longueur d'onde d'émission est fixe et prédéterminée en fonction du composant à mesurer, ladite au moins une source lumineuse étant associée aux moyens de détection (18c).

6. Appareil de mesure selon les revendications 1 et 2, **caractérisé en ce que** les moyens pour effectuer des analyses par colorimétrie comportent au moins deux sources lumineuses (22) de longueurs d'onde d'émission différentes, chaque longueur d'onde étant fixe et prédéterminée pour la mesure de la couleur du vin, lesdites sources lumineuses étant associées aux moyens de détection (18d).

7. Appareil de mesure selon les revendications 1 à 6, **caractérisé en ce que** l'échantillon à mesurer est disposé entre les sources lumineuses (20, 21, 22) et les moyens de détection (18c, 18d).

8. Appareil de mesure selon la revendication 7, **caractérisé en ce que** l'échantillon à mesurer est disposé dans un logement (16) ménagé au centre d'un puits de mesure (13) de base carrée, ce puits étant pourvu sur deux de ses faces adjacentes (13a, 13b) d'un conduit de lumière (17a, 17b) ménagé entre ladite face et le logement (16), l'axe longitudinal dudit conduit de lumière étant perpendiculaire au plan de la face dans laquelle il débouche, les moyens de détection (18c, 18d) étant incorporés respectivement dans les deux autres faces (13c, 13d) et disposés dans l'alignement des conduits de lumière (17a, 17b).

9. Appareil de mesure selon la revendication 2, **caractérisé en ce que** les moyens de détection (18c, 18d) sont des photodiodes au silicium.

10. Appareil de mesure selon les revendications 2 et 8, **caractérisé en ce que** les moyens de détection (18c, 18d) sont disposés à la sortie des conduits de lumière (17a, 17b) et à 90 degrés l'un de l'autre par rapport à l'échantillon à contrôler afin de permettre une mesure de la turbidité en mode axial et en quadrature.

11. Appareil de mesure selon les revendications 2, 5, 6 et 8, **caractérisé en ce que** la source lumineuse (20) et la au moins une source lumineuse (21) d'une part et les sources lumineuses (22) d'autre part sont disposées respectivement à l'entrée des conduits de lumière (17a, 17b) et à 90 degrés les unes des autres par rapport à l'échantillon à contrôler.

12. Appareil de mesure selon les revendications 2, 5 et 6, **caractérisé en ce que** les sources lumineuses (20, 21, 22) sont des diodes photoluminescentes.

13. Appareil de mesure selon les revendications 2, 5 et 6, **caractérisé en ce qu'**il comporte des moyens de traitement (19) des signaux émis par les moyens de détection (18c, 18d), lesdits moyens de traitement étant agencés pour rechercher les valeurs extrêmes mesurées par lesdits moyens de détection (18c, 18d) et associés à des moyens d'affichage (14) de ces valeurs mesurées.

14. Appareil de mesure selon la revendication 1, **caractérisé en ce que** les composants du moût dont la teneur est contrôlée sont le fer, le glycérol, l'acide tartrique et l'acétaldéhyde.

15. Appareil de mesure selon les revendications 2, 5 et 6, **caractérisé en ce qu'**il comporte des moyens agencés pour modifier l'intensité lumineuse des sources lumineuses (20, 21, 22) en fonction des valeurs à mesurer.

## Claims

1. Measuring apparatus (10) for measuring physical parameters of a grape must and wine obtained using said must and comprising means for measuring turbidity of a sample of said must by nephelemetry and further means for carrying out at least one analysis by colorimetry, in order to check the content of said sample of at least one predefined component and the colour of the wine obtained after fermentation of said must, **characterised in that** said means for carrying out the analysis by colorimetry are combined with means for carrying out a measurement of turbidity and are housed in the same easy-to-transport case (11).

2. Measuring apparatus according to claim 1, **characterised in that** the means for performing the measurement of turbidity comprise a light source (20) associated with the detection means (18c, 18d).

3. Measuring apparatus according to claim 2, **characterised in that** the light source (20) is a source of white light.

4. Measuring apparatus according to claim 2, **characterised in that** the light source (20) is a monochromatic light source.

5. Measuring apparatus according to claims 1 and 2, **characterised in that** the means for performing the analyses by colorimetry comprise at least one light source (20) whose emission wavelength is fixed and predetermined as a function of the compound to be measured, said at least one light source being combined with the detection means (18c).

6. Measuring apparatus according to claims 1 and 2, **characterised in that** the means for carrying out the analyses by colorimetry comprise at least two light sources (22) having different emission wavelengths, each wavelength being fixed and predetermined for measurement of the colour of the wine, said light sources being combined with the detection means (18d).

7. Measuring apparatus according to claims 1 to 6, **characterised in that** the sample to be measured is disposed between the light sources (20, 21, 22) and the detection means (18c, 18d).

8. Measuring apparatus according to claim 7, **characterised in that** the sample to be measured is disposed in a compartment (16) housed at the centre of a measuring well (13) having a square base, said well being provided on two of its adjacent faces (13a, 13b) with a light guide (17a, 17b) housed between said housing face (16), the longitudinal axis of said light guide being perpendicular to the plane of said face into which it opens, the detection means (18c, 18d) being incorporated, respectively, in the two other faces (13c, 13d) and disposed in alignment with the light guides (17a, 17b).

9. Measuring apparatus according to claim 2, **characterised in that** the detection means (18c, 18d) are silicon photodiodes.

10. Measuring apparatus according to claims 2 and 8, **characterised in that** the detection means (18c, 18d) are arranged at the exit of the light guides (17a, 17b) and at 90 degrees to each other relative to the sample to be tested, in order to allow a turbidity measurement axially and at right angles.

11. Measuring apparatus according to claims 2, 5, 6 and 8, **characterised in that** the light source (20) and the at least one light source (21) of one part and the light sources (22) of the other part are arranged, respectively at the entry of the light guides (17a, 17b) and at 90 degrees to each other with respect to the sample to be tested.

12. Measuring apparatus according to claims 2, 5 and 6, **characterised in that** the light sources (20, 21, 22) are photoluminescent diodes.

13. Measuring apparatus according to claims 2, 5 and 6, **characterised in that** it comprises a processing means (19) for signals emitted by the detection means (18c, 18d), said processing means being organised for seeking the end values measured by said detection means (18c, 18d) and combined with displaying means (14) for said measured values.

14. Measuring apparatus according to claim 1, **characterised in that** the compounds of the must whose content is being tested are iron, glycerol, tartaric acid and acetaldehyde.

15. Measuring apparatus according to claims 2, 5 and 8, **characterised in that** it comprises means operated for modifying the light intensity from the light sources (20, 21, 22) based on the values to be measured.

## Patentansprüche

1. Gerät (10) zum Messen physikalischer Parameter eines Traubenmostes und von aus diesem Traubenmost erhaltenem Wein, welches Einrichtungen zur Durchführung von Messungen des Trübungsgrades eines Musters des Mostes durch Trübungsmessung, und Einrichtungen zur Durchführung mindestens einer Analyse durch Farbmessung umfasst, um den Gehalt dieses Musters an mindestens einem zuvor festgelegten Bestandteil und die nach der Fermentation des Mostes erhaltene Farbe des Weines zu kontrollieren,
**dadurch gekennzeichnet, dass**
die Einrichtungen zur Durchführung einer Analyse durch Farbmessung mit Einrichtungen zur Durchführung einer Messung des Trübungsgrades verbunden und in demselben Gehäuse (11) untergebracht sind, welches leicht transportierbar ist.

2. Messgerät nach Anspruch 1,
**dadurch gekennzeichnet, dass**
dass die Einrichtungen zur Durchführung der Messung des Trübungsgrades eine mit Erkennungseinrichtungen (18c,18d) verbundene Lichtquelle (20) umfassen.

3. Abbruchverfahren nach Anspruch 2,
**dadurch gekennzeichnet, dass**
die Lichtquelle (20) eine weiße Lichtquelle ist.

4. Abbruchverfahren nach Anspruch 2,
**dadurch gekennzeichnet, dass**
die Lichtquelle (20) eine monochromatische Lichtquelle ist.

5. Messgerät nach den Ansprüchen 1 und 2,
**dadurch gekennzeichnet, dass**
die Einrichtungen zur Durchführung von Analysen durch Farbmessung mindestens eine Lichtquelle (21) umfassen, deren Ausgabewellenlänge fest und in Abhängigkeit von dem zu messenden Bestandteil zuvor festgelegt ist, wobei die mindestens eine Lichtquelle mit Erkennungseinrichtungen (18c) verbunden ist.

6. Messgerät nach den Ansprüchen 1 und 2,
**dadurch gekennzeichnet, dass**
die Einrichtungen zur Durchführung von Analysen durch Farbmessung mindestens zwei Lichtquellen (22) mit unterschiedlichen Ausgabewellenlängen umfassen, wobei jede Wellenlänge fest und für die Messung der Farbe des Weines zuvor festgelegt ist, wobei die Lichtquellen mit Erkennungseinrichtungen (18d) verbunden sind.

7. Messgerät nach den Ansprüchen 1 bis 6,
**dadurch gekennzeichnet, dass**
das zu messende Muster zwischen den Lichtquellen (20,21,22) und den Erkennungseinrichtungen (18c,18d) angeordnet ist.

8. Messgerät nach Anspruch 7,
**dadurch gekennzeichnet, dass**
das zu messende Muster in einer Aufnahme (16) angeordnet ist, die in den Mittelpunkt eines Messschachtes (13) mit quadratischer Basis eingearbeitet ist, wobei der Schacht auf zwei seiner aneinander angrenzenden Flächen (13a,13b) mit einer zwischen der Fläche und der Aufnahme (16) eingearbeiteten Lichtleitung (17a,17b) versehen ist, wobei die Längsachse der Lichtleitung senkrecht zu der Ebene der Seite verläuft, in die sie einmündet, wobei die Erkennungseinrichtungen (18c,18d) jeweils in die zwei anderen Flächen (13c,13d) integriert und in der Ausrichtung der Lichtleitungen (17a,17b) angeordnet sind.

9. Messgerät nach Anspruch 2,
**dadurch gekennzeichnet, dass**
die Erkennungseinrichtungen (18c,18d) Fotodioden aus Silizium sind.

10. Messgerät nach den Ansprüchen 2 und 8,
**dadurch gekennzeichnet, dass**
die Erkennungseinrichtungen (18c,18d) an dem Ausgang der Lichtleitungen (17a,17b) und mit 90 Grad im Verhältnis zu dem Muster angeordnet sind, um eine Messung des Trübungsgrades im Axial- und im Quadraturmodus um 90 Grad phasenverschoben zu ermöglichen.

11. Messgerät nach den Ansprüchen 2, 5, 6 und 8,
**dadurch gekennzeichnet, dass**
die Lichtquelle (20) und mindestens eine Lichtquelle (21) einerseits und die Lichtquellen (22) andererseits jeweils am Eingang der Lichtleitungen (17a,17b) und 90 Grad voneinander im Verhältnis zu dem zu kontrollierenden Muster entfernt sind.

12. Messgerät nach den Ansprüchen 2, 5 und 6,
**dadurch gekennzeichnet, dass**
die Lichtquellen (20,21,22) Fotolumineszenzdioden sind.

13. Messgerät nach den Ansprüchen 2, 5 und 6,
**dadurch gekennzeichnet, dass**
es Einrichtungen (19) zur Behandlung von durch die Erkennungseinrichtungen (18c,18d) ausgegebenen Signalen umfasst, wobei die Behandlungseinrichtungen angeordnet sind, um die von den Erkennungseinrichtungen (18c,18d) gemessenen Extremwerte zu ermitteln, die mit Anzeigeeinrichtungen (14) für die gemessenen Werte verbunden sind.

14. Messgerät nach Anspruch 1,
**dadurch gekennzeichnet, dass**
die Bestandteile des Mostes, deren Gehalt kontrolliert wird, Eisen, Glyzerin, Weinsäure und Azetaldehyd sind.

15. Messgerät nach den Ansprüchen 2, 5 und 6,
**dadurch gekennzeichnet, dass**
es Einrichtungen umfasst, die angeordnet sind, um die Lichtintensität der Lichtquellen (20,21,22) in Abhängigkeit von den zu messenden Werten zu verändern.
